# EUROPÄISCHE PATENTSCHRIFT

(11) **EP 0 951 893 B1**
(45) Veröffentlichungstag und Bekanntmachung des Hinweises auf die Patenterteilung: **19.05.2004**
(21) Anmeldenummer: 99105120.2
(22) Anmeldetag: 25.03.1999
(51) Int. Cl.: A61K 6/02, A61K 6/00

(54) **Antibakteriell wirksames Mittel zum Füllen von Wurzelkanälen und Herstellungsverfahren**
Antibacterial filling for root canals and method of manufacturing
Produit antibactérien pour l'obturation des canaux des racines dentaires et son procédé de préparation

(30) Priorität: 27.03.1998 DE 19813686
(43) Veröffentlichungstag der Anmeldung: 27.10.1999
(73) Patentinhaber: Coltène/Whaledent GmbH + Co. KG, 89129 Langenau (DE)
(72) Erfinder: Mannschedel, Werner, 89129 Langenau (DE)
(74) Vertreter: Forstmeyer, Dietmar, Dr. rer. nat., Dipl.-Chem.

(56) Entgegenhaltungen:
- EP-A- 0 388 220
- EP-A- 0 613 685
- WO-A-89/10736
- DE-A- 2 338 031

## Beschreibung

Die vorliegende Erfindung betrifft ein antibakteriell wirksames Mittel zum Füllen von Wurzelkanälen des Menschen oder eines Tieres. Sie betrifft ferner ein Herstellungsverfahren für das antibakteriell wirksame Mittel und dessen Verwendung zum Füllen von Wurzelkanälen.

Zur Therapie einer Erkrankung, die als Pulpitis bekannt ist, wird die erkrankte Pulpa mechanisch aus dem Wurzelkanal entfernt, der Wurzelkanal gereinigt und ausgebohrt, mit einem elastisch-plastischen Element oder einem anderen Füllmaterial gefüllt und danach versiegelt. Zum Stand der Technik sei beispielsweise auf Friedman et al. in J. Dent. Res., 54 (1975) 921-925, Briseno in Philipp J., 2, 90, 65-73 und die US-A-4 632 977 verwiesen. Briseno beschreibt als Wurzelkanalfüllmaterialien unter anderem halbfeste Zemente auf Kunstharzbasis, Zinkoxid-Eugenol-Basis, Calciumhydroxidbasis und Glas-Iononomerbasis. Die US-A-4 632 977 schlägt Füllmaterialien auf Trans-Poly-Isopren-Basis vor, beispielsweise auf Basis von Guttapercha oder Balata.

Im Handel sind Guttapercha-Spitzen erhältlich, deren Standardzusammensetzung 20 Gew.-% Guttapercha als Matrix, 60 bis 75 Gew.-% Zinkoxid als Füllstoff, 1 bis 17 Gew.-% Schwermetallsulfate als Röntgenkontrastmittel und 3 bis 4 Gew.-% Wachse und Harze als Weichmacher vorsieht. Dieses bekannte Füllmaterial ist im Wurzelkanal inert und reagiert demgemäß nicht mit dem Körpergewebe.

Bei der Füllung eines Wurzelkanals mit einem bekannten inerten Füllmaterial ist es jedoch nicht ausgeschlossen, daß es beispielsweise durch im Kanal verbliebene Keime nach der Füllung langsam zu einem entzündlichen Prozeß kommt, der eine erneute Behandlung erforderlich macht, die häufig zum vollständigen Zahnverlust führt.

Bisher war es daher üblich, Antibiotika oder Calciumhydroxid enthaltende Mittel einzusetzen. Antibiotika weisen jedoch eine Vielzahl von Nebenwirkungen auf, führen z.B. zur Bildung resistenter Bakterienstämme und werden insbesondere für Kinder in zunehmendem Maße abgelehnt.

Der Einsatz von Calciumhydroxid führt in einer Vielzahl von Fällen zum Erfolg, es ist jedoch in jüngerer Zeit festgestellt worden, daß einige Bakterien wie Streptococcus micros und Enterococcus faecalis auch bei einem pH-Wert von 11 beständig sind und somit nicht durch den Einsatz von Calciumhydroxid vollständig abgetötet werden können.

Ferner ist aus den US-Patenten 1 754 577 und 5 648 403 bekannt, Wurzelkanäle mit einem Träger zu verschließen, der Jod-haltige Verbindungen enthält. Da aber erkannt wurde, daß eine kontinuierliche Abgabe von Jod an den menschlichen Organismus zu schädlichen Nebenwirkungen führen kann und Jodverbindungen außerdem einen unansprechenden Geruch aufweisen, ist schon länger gefordert worden, auf derartige Jod-haltige Präparate zu verzichten.

Es werden auch Jod-haltige und Chlorhexidin-haltige Lösungen zur Spülung der Mundhöhle insbesondere vor, während und nach chirurgischen Eingriffen offenbart, vgl. US 4 738 840, US 3 932 607, EP 94 102 340.0, RU 200 88 42, SU 165 0 138, JP 622 65 225 oder Mittel, die antibakterielle Verbindungen in einem biologisch abbaubaren oder löslichen Träger enthalten, vgl. EP 90 302 837.1 und WO 89/10 736.

Gegenüber diesem Stand der Technik ist es eine Aufgabe der vorliegenden Erfindung, ein entzündungshemmendes Mittel zum Füllen von Wurzelkanälen des Menschen oder eines Tieres vorzusehen, das leicht und einfach hergestellt und verarbeitet werden kann und nicht mit den angesprochenen negativen Wirkungen des Stands der Technik belastet ist. Ferner ist es eine Aufgabe der vorliegenden Erfindung, ein Verfahren zur Herstellung eines derartigen Mittels vorzusehen.

Diese Aufgabe wird erfindungsgemäß durch ein Mittel zum Füllen von Wurzelkanälen des Menschen oder Tieres gelöst, das mindestens ein Chlorhexidinderivat von mindestens einer Carbonsäure, einen oder mehrere Träger auf Trans-Poly-Isopren-Basis, Guttapercha-Basis, Balata-Basis, Silikonbasis, Kautschukbasis, Acrylatbasis oder Gemische davon, gegebenenfalls einen oder mehrere Füllstoffe und ein oder mehrere Röntgenkontrastmittel enthält.

Das erfindungsgemäße Mittel kann z.B. in Form einer temporären Füllung oder auch als permanente Füllung von Wurzelkanälen des Menschen oder eines Tieres eingesetzt werden.

Insbesondere läßt sich das erfindungsgemäße antibakteriell wirksame Mittel in vorteilhafter Weise als Pulver oder als Flüssigkeit in einen Träger einwalzen, was den Herstellungsprozeß wesentlich vereinfacht. In alternativer Weise kann ein Träger, z.B. eine Guttaperchaspitze, mit dem/ den antibakteriell wirksamen Mittel(n) überzogen bzw. lackiert werden.

Der Träger kann dabei durch mindestens 80 Gew.-% Trans-Poly-Isopren gekennzeichnet sein.

Das erfindungsgemäße Mittel kann in Form einer Guttapercha-Spitze vorliegen und temporär, z.B. wenige Tage, über einen längeren Zeitraum oder permanent im Wurzelkanal verbleiben.

Als Füllstoff kann z.B. Zinkoxid eingesetzt werden, das in Form eines Gemisches mit einem oder mehreren üblichen anderen Füllstoffen, wie z.B. Siliciumdioxid, Aluminiumoxid, Calciumhydroxid oder als alleiniger Füllstoff vorgesehen werden kann.

Zusätzlich kann das erfindungsgemäße Mittel einen oder mehrere weitere pharmazeutische Wirkstoffe, insbesondere einen in wässerigem Medium lösbaren oder dispergierbaren Wirkstoff und ein oder mehrere übliche Wachse, Harze oder sonstige Hilfsstoffe wie Tenside (z.B. Polyethylenglykole wie Dipluronic) umfassen.

Ein erfindungsgemäßes antibakteriell wirksames Mittel kann vorzugsweise durch
(a) bis zu 99 Gew.-% Träger,
(b) bis zu 99 Gew.-% Füllstoff,
(c) 0,01 bis 50 Gew.-% mindestens eines Chlorhexidinderivates von mindestens einer Carbonsäure,
(d) bis zu 70 Gew.-% Röntgenkontrastmittel, bezogen auf (a), (b) und (c),
(e) bis zu 20 Gew.-% Tenside, und
(f) gegebenenfalls zusätzliche übliche Komponenten
gekennzeichnet sein.

So kann das erfindungsgemäße Mittel beispielsweise durch
(a) bis zu 99 Gew.-% Guttapercha,
(b) bis zu 99 Gew.-% Zinkoxid als Füllstoff,
(c) bis zu 50 Gew.-% eines Chlorhexidinderivates von mindestens einer Carbonsäure,
(d) bis zu 70 Gew.-% Röntgenkontrastmittel, bezogen auf (a), (b) und (c),
(e) 0,01 bis 20 Gew.-% Tensid, und
(f) gegebenenfalls zusätzliche übliche Komponenten
gekennzeichnet sein.

Wie vorstehend erwähnt, weist das erfindungsgemäße Mittel den Vorteil auf, daß es sowohl als temporäre Füllung als auch als permanente Füllung von Wurzelkanälen eingesetzt werden kann: Insbesondere wenn Guttapercha als Träger eingesetzt wird, kommt es weder zu einem Auswaschen noch zu einem biologischen Abbau des Trägers. Im Gegensatz zu den löslichen oder biologisch abbaubaren Trägern des Stands der Technik können derartige Mittel also beliebig lange im Mund verbleiben, ohne daß es zu einer Re-Infektions des Wurzelkanals kommt.

Enthält das Mittel z.B. bezogen auf die Gesamtzusammensetzung etwa 5 Gew. % eines erfindungsgemäßen antibakteriellen Mittels wie Chlorhexidindiacetat oder Chlorhexidingluconat, das in dem Guttaperchastift homogen verteilt ist und wird das Mittel in einen Wurzelkanal eingebracht, so lösen sich etwa 5 % des Chlorhexidindervates, so daß die Gesamtmenge des Guttaperchastiftes um max. 0,25 Gew. % abnimmt.

Bei einer derartig geringen Abnahme treten keine Läsionen auf, die eine Re-Infektion des Wurzelkanals ermöglichen, zumal Guttapercha plastische Eigenschaften aufweist.

Das erfindungsgemäße Mittel kann im wesentlichen vier Komponenten aufweisen, die im folgenden noch näher erläutert werden sollen.

### Komponente 1

Die mechanischen Eigenschaften eines erfindungsgemäßen Mittels zum Füllen von Wurzelkanälen werden in erster Linie durch die Eigenschaften der Träger bestimmt, die beispielsweise 35 bis 60,0 Gew.-%, vorzugsweise 15 bis 30 Gew.-% des Mittels ausmachen. Die Träger sollten einerseits elastisch sein, damit sie bequem verarbeitet und leicht in den Wurzelkanal eingebracht werden können. Andererseits sollten sie plastische Eigenschaften aufweisen, damit der Wurzelkanal dauerhaft ausgefüllt werden kann, ohne daß ein Wandspalt verbleibt. Darüber hinaus müssen die Träger andere Komponenten, insbesondere zumindest ein erfindungsgemäßes antibakteriell wirksames Mittel und gegebenenfalls einen Füllstoff, leicht aufnehmen können. Für diese Zwecke hat sich ein Träger mit mindestens 80 Gew.-% Trans-Poly-Isopren als vorteilhaft erwiesen. Als Beispiel kann Guttapercha angeführt werden, bei dem es sich um einen Träger auf natürlicher Basis handelt, dessen Hauptbestandteil Trans-Poly-Isopren ist. Selbstverständlich sind auch andere Trans-Poly-Isoprene einsetzbar, wie Balata, auch synthetische Träger auf Isopren-Basis, auf Silikon-, Kautschukbasis oder auf Acrylatbasis oder Abkömmlinge der genannten Materialien. Bei den aus dem Stand der Technik bekannten gelartigen Mitteln ist es schwierig, eine Einführung bis in den Apex zu erreichen und die notwendigerweise temporäre Einlage später wieder zu entfernen.

### Komponente 2

Das erfindungsgemäße Mittel ist dadurch gekennzeichnet, daß es als antibakteriell wirksames Mittel, mindestens ein Chlorhexidin (1,6-Di-(4-chlorophenyldiguanido)-hexan) Derivat mindestens einer Carbonsäure, vorzugsweise von einer oder zwei Carbonsäuren, enthält. Dieses antibakteriell wirksame Mittel liegt beispielsweise in einer Menge von 0,01 bis 50 Gew.-%, vorzugsweise von 0,01 bis 25 Gew.-%, vorzugsweise von 0,1 bis 15 Gew.-%, stärker bevorzugt von 0,2 bis 10 Gew.-%, noch stärker bevorzugt von 0,3 bis 5 Gew.-%, und am stärksten bevorzugt von 0,5 bis 2,0 Gew.-%, bezogen auf das gesamte Mittel vor. Bei den Chlorhexidinderivaten handelt es sich um gewebe- bzw. körperverträgliche Stoffe, die als Paste, wäßrige Lösung oder Pulver eingesetzt werden können. Z.B. können sie mit dem Träger gemischt und mit weiteren Komponenten, wie einem oder mehreren Füllstoffen, compoundiert werden. Derivate von Chlorhexidin von mindestens einer Carbonsäure sind zum Beispiel Chlorhexidingluconate, Chlorhexidindiacetate, wobei Chlorhexidindiacetat bevorzugt wird.

Das bzw. die antibakteriell wirksamen Mittel lassen sich zum Beispiel als Pulver oder als Flüssigkeit bzw. Lösung in einen Träger wie Guttapercha einwalzen, wodurch das Herstellungsverfahren recht einfach gestaltet werden kann.

Gegenüber der alleinigen Verwendung von Calciumhydroxid weist das erfindungsgemäße Mittel den zusätzlichen Vorteil eines anderen Wirkungsspektrums auf, zumal keine Resistenzentwicklung auftritt, so daß seine Akzeptanz gegenüber Antibiotika deutlich verbessert ist.

Ferner kann das Mittel, im Gegensatz zu den Calciumhydroxid enthaltenden Zusammensetzungen des Standes der Technik, permanent im Zahn belassen werden, da bei den zusammensetzungen des Standes der Technik große Mengen an Calciumhydroxid ausgewaschen werden, so daß es dort bei permanentem Einsatz zu unakzeptablen Läsionen kommen würde.

### Komponente 3

Als Füllstoffe kommen übliche Füllstoffe, insbesondere Zinkoxid, Siliciumdioxid, Aluminiumoxid und Calciumhydroxid allein oder in Kombination in an sich üblichen Mengen zum Einsatz, wobei Calciumhydroxid insbesondere bei temporären Füllungen eingesetzt werden kann. Diese Komponenten kommen in einer Menge von bis zu 99 Gew.-%, vorzugsweise in einer Menge von 10 bis 70 Gew.-%, noch stärker bevorzugt in einer Menge von 15 bis 40 Gew.-%, bezogen auf die Summe der Gewichte aller Komponenten des Mittels, zum Einsatz.

### Komponente 4

Als weitere, allerdings fakultative, wenn auch übliche Komponente kann ein Röntgenkontrastmittel vorgesehen werden, das aus der Gruppe der Zink-, Ytterbium-, Yttrium-, Gadolinium-, Zirkonium-, Strontium-, Wolfram-, Tantal-, Niob-, Barium-, Wismut-, Molybdän- und Lanthanpulver, pulvrigen Legierungen davon, Oxiden, Fluoriden, Sulfaten, Carbonaten, Wolframaten und Carbiden davon ausgewählt ist. Diese Komponente kann in einer Menge von bis zu 70 Gew.-%, vorzugsweise von 10 bis 60 Gew.-%, stärker bevorzugt von 15 bis 40 Gew.-%, bezogen auf die Summe der Gewichte der Komponenten 1, 2 und 3, vorgesehen werden.

Gemäß einer weiteren Ausführungsform der Erfindung wird schließlich ein Verfahren zum Herstellen eines erfindungsgemäßen Mittels zum Füllen von Wurzelkanälen vorgesehen, wobei man
(a) einen Film eines Trägers auf Isopren-Basis bildet,
(b) in den Film des Trägers gegebenenfalls einen oder mehrere Füllstoffe sowie gegebenenfalls ein oder mehrere Röntgenkontrastmittel, Tenside und weitere fakultative übliche Hilfsmittel vorzugsweise in Pulverform oder nicht zu hoch viskoser Form einwalzt,
(c) den compoundierten Film zerkleinert und - vorzugsweise in an sich üblicher Weise - extrudiert,
(d) das Extrudat zerschneidet und den anfallenden Schnitt zu elastisch-plastischen Elementen rollt, die zum Füllen von Wurzelkanälen des Menschen oder eines Tieres geeignet sind,
(e) in Stufe (b) in den Film des Trägers mindestens ein erfindungsgemäßes antibakterielles Mittel einwalzt, und/oder
(f) den Film gemäß (b) oder (c) oder das Element gemäß (d) mit einem mindestens ein antibakterielles Mittel enthaltenden Lack oder Pulver überzieht bzw. beschichtet.

Vorzugsweise steigt die Temperatur bei der Extrusion von 50°C auf 120°C, besonders bevorzugt von 60°C auf 110°C, wodurch ein einheitliches und keimfreies Produkt erhalten wird.

Nachstehend wird die Erfindung durch ein Beispiel näher erläutert.

### Beispiel

Zur Herstellung von Guttaperchaspitzen wurde Zinkoxid enthaltender Guttapercha als Matrix zunächst zwischen einer Trägerwalze und einer Andruckwalze verwalzt, so daß sich die Guttapercha als dünner Film bzw. fellartig um die Trägerwalze legte. Danach wurden 8 Gew.-% Chlorhexidindiacetat und 92 Gew.-% der Guttapercha-Zinkoxidmatrix in den gebildeten Film eingewalzt, der Film von der Trägerwalze abgeschält, der abgeschälte Film zerkleinert und bei einer Temperatur, die von 60°C auf 110°C ansteigt, zu einem dünnen drahtartigen Strang extrudiert. Dieser Strang wurde danach zerschnitten und rolliert.

## Patentansprüche

1. Mittel zum Füllen von Wurzelkanälen des Menschen oder eines Tieres, das
a) mindestens ein Chlorhexidinderivat von mindestens einer Carbonsäure,
b) einen oder mehrere Träger auf Trans-Poly-Isopren-Basis, Guttapercha-Basis, Balata-Basis, Silikonbasis, Kautschukbasis, Acrylatbasis oder Gemische davon,
c) gegebenenfalls einen oder mehrere Füllstoffe und
d) gegebenenfalls ein oder mehrere Röntgenkontraststoffe enthält.

2. Mittel nach Anspruch 1, ***gekennzeichnet* durch** einen Träger mit mindestens 80 Gew.-% Trans-Poly-Isopren.

3. Mittel nach einem der vorhergehenden Ansprüche in Form einer Guttapercha-Spitze.

4. Mittel nach einem der vorhergehenden Ansprüche, wobei der Füllstoff Zinkoxid, Siliciumdioxid, Aluminiumoxid, Calciumhydroxid oder ein Gemisch davon ist.

5. Mittel nach einem der vorhergehenden Ansprüche, ***gekennzeichnet* durch** einen Träger auf Guttaperchabasis.

6. Mittel nach einem der vorhergehenden Ansprüche, ***gekennzeichnet* durch** einen zusätzlichen Gehalt an einem oder mehreren üblichen Wachsen und/oder Harzen.

7. Mittel nach einem der vorhergehenden Ansprüche, ***gekennzeichnet* durch** einen zusätzlichen Gehalt an einem üblichen Tensid.

8. Mittel nach einem der vorhergehenden Ansprüche, **dadurch *gekennzeichnet,* daß** das Röntgenkontrastmittel aus der Gruppe der Zink-, Ytterbium-, Yttrium-, Gadolinium-, Zirkonium-, Strontium-, Wolfram-, Tantal-, Niob-, Barium-, Wismut-, Molybdän- und Lanthanpulver, pulvrigen Legierungen davon, Oxiden, Fluoriden, Sulfaten, Carbonaten, Wolframaten und Carbiden davon ausgewählt ist.

9. Mittel nach einem der vorhergehenden Ansprüche, ***gekennzeichnet* durch** einen zusätzlichen Gehalt an einem üblichen pharmazeutischen Wirkstoff, insbesondere einen in wässerigem Medium lösbaren oder dispergierbaren Wirkstoff.

10. Mittel nach einem der vorhergehenden Ansprüche, ***gekennzeichnet* durch**
(a) bis zu 99,0 Gew.-% Träger,
(b) bis zu 99 Gew.-% Füllstoff,
(c) 0,01 bis 50 Gew.-% antibakteriell wirksames Mittel,
(d) bis zu 70 Gew.-% Röntgenkontrastmittel, bezogen auf (a), (b) und (c),
(e) bis zu 20 Gew.-% Tenside, und
(f) gegebenenfalls zusätzliche übliche Komponenten.

11. Mittel nach Anspruch 10, ***gekennzeichnet* durch**
(a) bis zu 99 Gew.-% Guttapercha,
(b) bis zu 99 Gew.-% Zinkoxid als Füllstoff,
(c) bis zu 50 Gew.-% antibakteriell wirksames Mittel,
(d) bis zu 70 Gew.-% Röntgenkontrastmittel, bezogen auf (a), (b) und (c),
(e) 0,01 bis 20 Gew.-% Tensid, und
(f) gegebenenfalls zusätzliche übliche Komponenten.

12. Verfahren zum Herstellen eines Mittels nach einem der vorhergehenden Ansprüche, **dadurch *gekennzeichnet*, daß** man
(a) einen Film eines Trägers auf Isopren-Basis bildet,
(b) in den Film des Trägers gegebenenfalls einen oder mehrere Füllstoffe sowie gegebenenfalls ein oder mehrere Röntgenkontrastmittel, Tenside und weitere fakultative übliche Hilfsmittel einwalzt,
(c) den Film zu einem entsprechenden Mittel formt,
(d) in Stufe (b) in den Film des Trägers mindestens ein antibakterielles Mittel einwalzt, und/oder
(e) den Film gemäß (b) oder (c) mit einem mindestens ein antibakterielles Mittel enthaltenden Lack oder Pulver überzieht bzw. beschichtet.

13. Verfahren nach Anspruch 12, **dadurch gekennzeichnet, daß** der Film nach Schritt (b) zerkleinert und extrudiert, das Extrudat zerschnitten und der anfallende Schnitt zu dem Mittel gerollt wird.

14. Verfahren nach Anspruch 13, **dadurch gekennzeichnet, daß** die Temperatur bei der Extrusion von 50° C auf 120°, vorzugsweise von 60° C auf 110° C ansteigt.

15. Verwendung eines Mittels nach einem der Ansprüche 1-11 zur Herstellung einer temporären Einlage von Wurzelkanälen.

16. Verwendung eines Mittels nach einem der Ansprüche 1-11 zur Herstellung einer permanenten Füllung von Wurzelkanälen.

## Claims

1. Composition for filling root canals in humans or animals, comprising
a) at least one chlorhexidine derivative of at least one carboxylic acid,
b) one or more carriers based on trans-polyisoprene, gutta-percha, balata, silicone, caoutchouc, acrylate, or mixtures thereof,
c) optionally one or more fillers, and
d) optionally one or more X-ray contrast substances.

2. Composition according to claim 1, **characterised by** a carrier comprising at least 80% by weight of trans-polyisoprene.

3. Composition according to any one of the preceding claims in the form of a gutta-percha point.

4. Composition according to any one of the preceding claims, wherein the filler is zinc oxide, silicon dioxide, aluminium oxide, calcium hydroxide or a mixture thereof.

5. Composition according to any one of the preceding claims, **characterised by** a carrier based on gutta-percha.

6. Composition according to any one of the preceding claims, **characterised in that** it additionally comprises one or more customary waxes and/or resins.

7. Composition according to any one of the preceding claims, **characterised in that** it additionally comprises a customary surfactant.

8. Composition according to any one of the preceding claims, **characterised in that** the X-ray contrast agent is selected from the group consisting of zinc, ytterbium, yttrium, gadolinium, zirconium, strontium, tungsten, tantalum, niobium, barium, bismuth, molybdenum and lanthanum powders, powdered alloys thereof, oxides, fluorides, sulphates, carbonates, tungstates and carbides thereof.

9. Composition according to any one of the preceding claims, **characterised in that** it additionally comprises a customary pharmaceutical active ingredient, especially an active ingredient that is soluble or dispersible in an aqueous medium.

10. Composition according to any one of the preceding claims, **characterised by**
(a) up to 99.0% by weight of carrier,
(b) up to 99% by weight of filler,
(c) from 0.01 to 50% by weight of antibacterially active composition,
(d) up to 70% by weight of X-ray contrast agent, based on (a), (b) and (c),
(e) up to 20% by weight of surfactants, and
(f) optionally additional customary components.

11. Composition according to claim 10, **characterised by**
(a) up to 99% by weight of gutta-percha,
(b) up to 99% by weight of zinc oxide as filler,
(c) up to 50% by weight of antibacterially active composition,
(d0 up to 70% by weight of X-ray contrast agent, based on (a), (b) and (c),
(e) from 0.01 to 20% by weight of surfactant, and
(f) optionally additional customary components.

12. Method of preparing a composition according to any one of the preceding claims, **characterised in that**
(a) a film of an isoprene-based carrier is formed,
(b) there are rolled into the carrier film optionally one or more fillers and optionally one or more X-ray contrast agents, surfactants and further optional customary auxiliaries,
(c) the film is formed into an appropriate means,
(d) in step (b) at least one antibacterial composition is rolled into the carrier film, and/or
(e) the film according to (b) or (c) is covered or coated with a surface-coating or powder comprising at least one antibacterial composition.

13. Method according to claim 12, **characterised in that** the film according to step (b) is comminuted and extruded, the extruded product is cut up and each resulting cut piece is rolled to form the means.

14. Method according to claim 13, **characterised in that** the temperature during extrusion rises from 50°C to 120°C, preferably from 60°C to 110°C.

15. Use of a composition according to any one of claims 1 to 11 for the preparation of a temporary insert in root canals.

16. Use of a composition according to any one of claims 1 to 11 for the preparation of a permanent filling in root canals.

## Revendications

1. Produit pour l'obturation des canaux des racines dentaires de l'homme ou d'un animal, qui contient :
a) au moins un dérivé chlorhexidine d'au moins un acide carboxylique,
b) un ou plusieurs supports à base de trans-poly-isoprène, de gutta-percha, de balata, de silicone, de caoutchouc, d'acrylate ou de mélanges de ces derniers,
c) le cas échéant une ou plusieurs charges et
d) le cas échéant un ou plusieurs produits de contraste aux rayons X.

2. Produit selon la revendication 1, **caractérisé par** un support avec au moins 80 % en poids de trans-poly-isoprène.

3. Produit selon l'une quelconque des revendications précédentes sous la forme d'une pointe de gutta-percha.

4. Produit selon l'une quelconque des revendications précédentes, dans lequel la charge est de l'oxyde de zinc, du bioxyde de silicium, de l'oxyde d'aluminium, de l'hydroxyde de calcium ou un mélange de ces derniers.

5. Produit selon l'une quelconque des revendications précédentes, **caractérisé par** un support à base de gutta-percha.

6. Produit selon l'une quelconque des revendications précédentes, **caractérisé par** une teneur supplémentaire en une ou plusieurs cires et / ou résines usuelles.

7. Produit selon l'une quelconque des revendications précédentes, **caractérisé par** une teneur supplémentaire en un tensioactif usuel.

8. Produit selon l'une quelconque des revendications précédentes, **caractérisé en ce que** le produit de contraste aux rayons X est choisi parmi le groupe des poudres de zinc, d'ytterbium, d'yttrium, de gadolinium, de zirconium, de strontium, de tungstène, de tantale, de niobium, de baryum, de bismuth, de molybdène et de lanthane, parmi des alliages pulvérulents de ces derniers, parmi des oxydes, des fluorures, des sulfates, des carbonates, des tungstates et des carbures de ces derniers.

9. Produit selon l'une quelconque des revendications précédentes, **caractérisé par** une teneur supplémentaire en un agent activé pharmaceutique usuel, en particulier un qui est soluble en milieu aqueux ou peut y être dispersé.

10. Produit selon l'une quelconque des revendications précédentes **caractérisé par**
(a) jusqu'à 99,0 % en poids de support,
(b) jusqu'à 99 % en poids de charge
(c) de 0,01 à 50 % en poids d'agent à effet antibactérien,
(d) jusqu'à 70 % en poids de produit de contraste aux rayons X, rapporté à (a), (b) et (c),
(e) jusqu'à 20 % en poids d'agents tensioactifs et
(f) le cas échéant des composants supplémentaires usuels.

11. Produit selon la revendication 10, **caractérisé par**
(a) jusqu'à 99 % en poids de gutta-percha,
(b) jusqu'à 99 % en poids d'oxyde de zinc comme charge,
(c) jusqu'à 50 % en poids d'agent à effet antibactérien,
(d) jusqu'à 70 % en poids de produit de contraste aux rayons X, rapporté à (a), (b) et (c),
(e) de 0,01 à 50 % en poids d'agent tensioactif et
(f) le cas échéant des composants supplémentaires usuels.

12. Procédé de fabrication d'un produit selon l'une quelconque des revendications précédentes, **caractérisé en ce que**
(a) l'on forme un film d'un support à base d'isoprène,
(b) on presse le cas échéant au rouleau dans le film du support une ou plusieurs charges ainsi que le cas échéant un ou plusieurs produits de contraste aux rayons X, d'agents tensioactifs et d'autres agents auxiliaires facultatifs usuels,
(c) on forme le film en un milieu adéquat,
(d) a l'étape (b), on presse au rouleau dans le film du support au moins une composition antibactérien et / ou
(e) on recouvre, respectivement on revêt le film selon (b) ou (c) d'une revêtement de surface ou d'une poudre contenant au moins une composition antibactérien.

13. Procédé selon la revendication 12, **caractérisé en ce qu'**après l'étape (b), le film est pulvérise et extrudé, le produit extrudé est découpé et le résultat du découpage est roulé pour donner le produit.

14. Procédé selon la revendication 13, **caractérisé en ce que** la température augmente pendant l'extrusion de 50°C à 120°C, de préférence de 60°C à 110°C.

15. Utilisation d'un produit selon l'une quelconque des revendications 1 à 11 pour la fabrication d'un emplâtre temporaire de canaux de racines.

16. Utilisation d'un produit selon l'une quelconque des revendications 1 à 11 pour la fabrication d'une obturation permanente de canaux de racines.
